# EUROPEAN PATENT APPLICATION

(11) **EP 4 640 218 A1**
(43) Date of publication of application: **29.10.2025**
(21) Application number: 23905890.2
(22) Date of filing: 18.12.2023
(51) Int. Cl.: A61K 31/425, A61K 31/4439, A61P 35/00

(54) **USE OF SULTAM COMPOUND IN TREATING BILIARY TRACT CANCER**

(30) Priority: 19.12.2022 CN 202211632640; 02.08.2023 CN 202310971301
(71) Applicant: Chia Tai Tianqing Pharmaceutical Group Co., Ltd., Lianyungang, Jiangsu 222062 (CN)
(72) Inventor: LI, Jie, Lianyungang, Jiangsu 222062 (CN); YU, Ding, Lianyungang, Jiangsu 222062 (CN); YANG, Anqi, Lianyungang, Jiangsu 222062 (CN); LI, Xu, Lianyungang, Jiangsu 222062 (CN); WANG, Xunqiang, Lianyungang, Jiangsu 222062 (CN); ZHANG, Xiquan, Lianyungang, Jiangsu 222062 (CN)
(74) Representative: v. Bezold & Partner Patentanwälte - PartG mbB
(86) International application number: PCT/CN2023/139528
(87) International publication number: WO 2024/131727

(57) **Abstract**

The present disclosure relates to use of a sultam compound in treating biliary tract cancer, and specifically relates to use of a compound represented by formula I or a pharmaceutically acceptable salt thereof in treating biliary tract cancer. The compound represented by formula I or the pharmaceutically acceptable salt thereof of the present disclosure has good safety and anti-tumor activity.

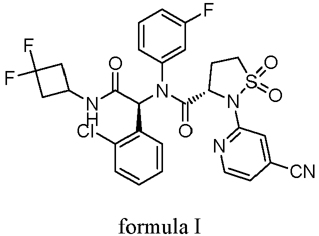

## Description

### CROSS-REFERENCE TO RELATED APPLICATION

The present application claims priority and benefit to the patent application Nos. 202211632640.5 and 202310971301.8 filed with the National Intellectual Property Administration, PRC on December 19, 2022 and August 02, 2023, respectively, which are incorporated herein by reference in their entirety.

### TECHNICAL FIELD

The present application pertains to the field of medical chemistry, and relates to use of a sultam compound for treating biliary tract cancer, and particularly to use of a compound of formula I or a pharmaceutically acceptable salt thereof for treating biliary tract cancer.

### BACKGROUND

IDH is known as isocitrate dehydrogenase, and is the most important key enzyme in the intracellular tricarboxylic acid cycle. IDH catalyzes the oxidative decarboxylation of isocitrate, producing 2-oxoglutarate (i.e., α-ketoglutarate). IDH has two distinct subtypes, one of which utilizes NAD(+) as the electron acceptor and the other NADP(+). Five IDHs have been reported, 3 of which are NAD(+)-dependent isocitrate dehydrogenases, which are located in the mitochondrial matrix; and two are NADP(+)-dependent isocitrate dehydrogenases, one of which is located in the mitochondria and the other in the cytoplasm.

According to the research, IDH mutations are found in various tumors (such as glioma, sarcoma, and acute myelocytic leukemia), and are located at arginine residues of the catalytic center (IDH1/R132H, IDH2/R140Q, and IDH2/R172K). In 2009, Bleeker *et al.* detected IDH1 mutations in 672 tumor samples of different origins and 84 different tumor cell lines and found that these mutations specifically and centrally occurred in gliomas (Bleeker et al., 2009. IDH1 mutations at residue p.R132(IDH1(R132)) occur frequently in high-grade gliomas but not in other solid tumors. Hum Mutat. 30: 7-11.). However, subsequent literature reports have shown that IDH1 mutations also exist in acute myelogenous leukemia, prostate cancer, paraganglioma, etc. (Green et al., 2010, Somatic mutations of IDH1 and IDH2 in the leukemic transformation of myeloproliferative neoplasms. N Engl J Med. 362: 369-370.). Bleeker *et al.* found that among the cases of IDH1 mutations, R132H accounted for 86.9%, with relatively small proportions of other types such as R132C, R132G, R132L, R132V, and R132S (Bleeker *et al.,* 2009).

WO2017162157 discloses a series of compounds that act as IDH1 inhibitors, and also specifically discloses a compound of formula I with the following structure:

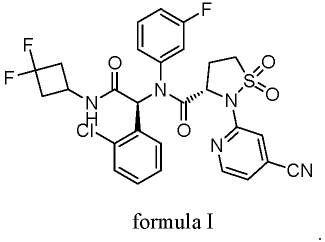

### SUMMARY

In one aspect, the present application provides a compound of formula I or a pharmaceutically acceptable salt thereof for use in treating biliary tract cancer in a patient,

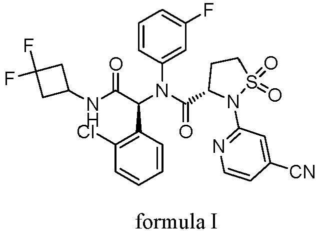

In one aspect, the present application provides a pharmaceutical composition for use in treating biliary tract cancer in a patient, the pharmaceutical composition comprising the compound of formula I or the pharmaceutically acceptable salt thereof.

In one aspect, the present application provides a method for treating biliary tract cancer in a patient, comprising administering to the patient an effective amount of the compound of formula I or the pharmaceutically acceptable salt thereof, or the pharmaceutical composition thereof described above.

In one aspect, the present application provides use of the compound of formula I or the pharmaceutically acceptable salt thereof, or the pharmaceutical composition thereof described above for preparing a medicament for treating biliary tract cancer in a patient.

In one aspect, the present application provides use of the compound of formula I or the pharmaceutically acceptable salt thereof, or the pharmaceutical composition thereof described above for treating biliary tract cancer in a patient. In one aspect, the present application provides a medicine for treating biliary tract cancer, comprising the compound of formula I or the pharmaceutically acceptable salt thereof described above as an active ingredient.

In some embodiments of the present application, the compound of formula I or the pharmaceutically acceptable salt thereof described herein is used as a single active agent. In some embodiments of the present application, the compound of formula I or the pharmaceutically acceptable salt thereof described herein is used as the sole active agent.

In some embodiments of the present application, the compound of formula I or the pharmaceutically acceptable salt thereof described herein may also be used in combination with an additional chemotherapeutic drug.

In some embodiments of the present application, the additional chemotherapeutic drug comprises a pyrimidine anti-tumor drug, a fluorouracil anti-tumor drug, a platinum-based anti-tumor drug, a paclitaxel anti-tumor drug, a tyrosine kinase inhibitor, a PD-1/PD-L1 inhibitor, a camptothecin anti-tumor drug, a BRAF inhibitor, or any combination thereof.

In some embodiments of the present application, the additional chemotherapeutic drug comprises gemcitabine; 5-fluorouracil; a combination of gemcitabine, cisplatin, and albumin paclitaxel; a combination of 5-fluorouracil and oxaliplatin; a combination of capecitabine and oxaliplatin; a combination of gemcitabine and capecitabine; a combination of gemcitabine and cisplatin; a combination of 5-fluorouracil and cisplatin; a combination of capecitabine and cisplatin; a combination of gemcitabine and oxaliplatin; a combination of gemcitabine, cisplatin, and tegafur-gimeracil-oteracil potassium; a combination of 5-fluorouracil and oxaliplatin; a combination of gemcitabine and albumin paclitaxel; entrectinib; larotrectinib; pembrolizumab; camrelizumab; tegafur-gimeracil-oteracil potassium; capecitabine; a combination of irinotecan and capecitabine; regorafenib; and a combination of dabrafenib and trametinib.

In some embodiments of the present application, the additional chemotherapeutic drug is a fluorouracil anti-tumor drug, a platinum-based anti-tumor drug, or a combination thereof.

In some embodiments of the present application, the additional chemotherapeutic drug is a combination of capecitabine and oxaliplatin.

In some embodiments of the present application, when the compound of formula I or the pharmaceutically acceptable salt thereof described herein is used in combination with an additional chemotherapeutic drug, the compound of formula I or the pharmaceutically acceptable salt thereof is administered in the same manner as when used as a single active agent.

In some embodiments of the present application, the compound of formula I or the pharmaceutically acceptable salt thereof described herein may be provided in the form of a pharmaceutical composition comprising a therapeutically effective amount of the compound of formula I or the pharmaceutically acceptable salt thereof.

In some embodiments of the present application, in the pharmaceutical composition, the compound of formula I or the pharmaceutically acceptable salt thereof is in a single dose or in multiple doses.

In some embodiments of the present application, the pharmaceutical composition is packaged in a kit, and the kit further comprises an instruction for treating biliary tract cancer in a patient with the compound of formula I or the pharmaceutically acceptable salt thereof or the pharmaceutical composition.

In some embodiments of the present application, the pharmaceutical composition comprises 100-1200 mg of the compound of formula I or the pharmaceutically acceptable salt thereof by weight of the compound of formula I.

In some embodiments of the present application, the pharmaceutical composition comprises 100 mg, 200 mg, 300 mg, 400 mg, 500 mg, 600 mg, 700 mg, 800 mg, 900 mg, 1000 mg, or a range formed by any of the above values of the compound of formula I or the pharmaceutically acceptable salt thereof by weight of the compound of formula I.

In some embodiments of the present application, the pharmaceutical composition comprises 100-900 mg, 200-900 mg, 200-800 mg, 200-600 mg, 400-900 mg, or 400-600 mg of the compound of formula I or the pharmaceutically acceptable salt thereof by weight of the compound of formula I.

In some embodiments of the present application, the pharmaceutical composition comprises 400-600 mg or 400-900 mg of the compound of formula I or the pharmaceutically acceptable salt thereof by weight of the compound of formula I.

In some embodiments of the present application, the pharmaceutical composition comprises 400 mg, 600 mg, or 900 mg of the compound of formula I or the pharmaceutically acceptable salt thereof by weight of the compound of formula I.

In some embodiments of the present application, the pharmaceutical composition comprises 400 mg or 600 mg of the compound of formula I or the pharmaceutically acceptable salt thereof by weight of the compound of formula I.

In some embodiments of the present application, the pharmaceutical composition comprises 600 mg of the compound of formula I or the pharmaceutically acceptable salt thereof by weight of the compound of formula I.

In some embodiments of the present application, the pharmaceutical composition of the compound of formula I or the pharmaceutically acceptable salt thereof is in a single-dose form or in a multi-dose form. In some embodiments of the present application, the pharmaceutical composition of the compound of formula I or the pharmaceutically acceptable salt thereof is in a multi-dose form.

In some embodiments of the present application, in the pharmaceutical composition, the content of the compound of formula I or the pharmaceutically acceptable salt thereof is a daily dose.

In some embodiments of the present application, in the pharmaceutical composition, the content of the compound of formula I or the pharmaceutically acceptable salt thereof is a once-daily dose.

In some embodiments of the present application, in the pharmaceutical composition, the content of the compound of formula I or the pharmaceutically acceptable salt thereof is a once-daily dose, with each dose being a single dose or multiple doses, typically multiple doses.

In some embodiments of the present application, in the pharmaceutical composition, the content of the compound of formula I or the pharmaceutically acceptable salt thereof is a once-daily dose, with each dose being a single dose or multiple doses, typically multiple doses, and the multiple doses are 400 mg, 600 mg, or 900 mg; preferably, the multiple doses are 400 mg or 600 mg; or preferably, the multiple doses are 600 mg.

In some embodiments of the present application, the pharmaceutical composition comprises the compound of formula I or the pharmaceutically acceptable salt thereof in a single dose of 50 mg and/or 200 mg by weight of the compound of formula I. Alternatively, the pharmaceutical composition is in the form of a single-administration formulation, and the pharmaceutical composition comprises 50 mg and/or 200 mg of the compound of formula I or the pharmaceutically acceptable salt thereof by weight of the compound of formula I. Alternatively, the pharmaceutical composition is in the form of a single-administration formulation, and the pharmaceutical composition comprises 50 mg and/or 200 mg or an integer multiple thereof (e.g., 100 mg, 150 mg, or 400 mg) of the compound of formula I or the pharmaceutically acceptable salt thereof by weight of the compound of formula I.

In some embodiments of the present application, the pharmaceutical composition comprises the compound of formula I or the pharmaceutically acceptable salt thereof in a single dose of 200 mg by weight of the compound of formula I. Alternatively, the pharmaceutical composition is in the form of a single-administration formulation, and the pharmaceutical composition comprises 200 mg of the compound of formula I or the pharmaceutically acceptable salt thereof by weight of the compound of formula I. Alternatively, the pharmaceutical composition is in the form of a single-administration formulation, and the pharmaceutical composition comprises 200 mg or an integer multiple thereof of the compound of formula I or the pharmaceutically acceptable salt thereof by weight of the compound of formula I.

In some embodiments of the present application, in the pharmaceutical composition, the content of the compound of formula I or the pharmaceutically acceptable salt thereof is a once-daily dose, with each dose being a single dose or multiple doses, typically multiple doses, and the multiple doses are 400 mg, 600 mg, or 900 mg; preferably, the multiple doses are 400 mg or 600 mg; or preferably, the multiple doses are 600 mg; the multiple doses consist of single doses of 200 mg of the compound of formula I or the pharmaceutically acceptable salt thereof by weight of the compound of formula I.

In some embodiments of the present application, every 2-6 weeks constitutes one treatment cycle. In some embodiments of the present application, every 28 days constitutes one treatment cycle. In some embodiments of the present application, every 21 days constitutes one treatment cycle.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 2.8 g-33.6 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 2.1 g-25.2 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 5.6 g-25.2 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 4.2 g-18.9 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 5.6 g-16.8 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 4.2 g-12.6 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 11.2 g-25.2 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 8.4 g-18.9 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 11.2 g-16.8 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 8.4 g-12.6 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 11.2 g, 16.8 g, or 25.2 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 8.4 g, 12.6 g, or 18.9 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 11.2 g or 16.8 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 8.4 g or 12.6 g by weight of the compound of formula I.

In some embodiments of the present application, the pharmaceutical composition is a formulation suitable for administration within a single treatment cycle (e.g., a 28-day treatment cycle), and the formulation comprises a pharmaceutical composition of the compound of formula I or the pharmaceutically acceptable salt thereof in a total dose of 16.8 g by weight of the compound of formula I. In some embodiments of the present application, the pharmaceutical composition is a formulation suitable for administration within a single treatment cycle (e.g., a 21-day treatment cycle), and the formulation comprises a pharmaceutical composition of the compound of formula I or the pharmaceutically acceptable salt thereof in a total dose of 12.6 g by weight of the compound of formula I. In another aspect, the present application also provides a kit for treating biliary tract cancer in a patient, comprising the compound of formula I or the pharmaceutically acceptable salt thereof, or the pharmaceutical composition described herein.

In another aspect, the present application also provides a method for treating biliary tract cancer in a patient, comprising administering to an individual in need thereof a therapeutically effective amount of the compound of formula I or the pharmaceutically acceptable salt thereof, or the pharmaceutical composition, for example, administering to an individual in need thereof a therapeutically effective amount of the pharmaceutical composition described above in the present application.

In another aspect, the present application also provides a therapy for treating an individual with biliary tract cancer, comprising administering separately to the individual a therapeutically effective amount of the compound of formula I or the pharmaceutically acceptable salt thereof, or the pharmaceutical composition, for example, administering separately to the individual a therapeutically effective amount of the pharmaceutical composition described above in the present application.

In another aspect, the present application also provides use of the compound of formula I or the pharmaceutically acceptable salt thereof, or the pharmaceutical composition for preparing a medicament for treating biliary tract cancer in a patient, for example, use of the pharmaceutical composition described above in the present application for preparing a medicament for treating biliary tract cancer in a patient. In some embodiments of the present application, the pharmaceutical composition is any of the pharmaceutical compositions described above in the present application.

In another aspect, the present application also provides use of the compound of formula I or the pharmaceutically acceptable salt thereof, or the pharmaceutical composition for treating biliary tract cancer in a patient, for example, use of the pharmaceutical composition described above in the present application for treating biliary tract cancer in a patient.

In some embodiments of the present application, in the method, the therapy, or the use, the definition of the compound of formula I or the pharmaceutically acceptable salt thereof is the same as that in the pharmaceutical composition described above, for example, in terms of content, dose, form of presence, form of packaging, or the like.

In some embodiments of the present application, in the method, the therapy, or the use, the compound of formula I or the pharmaceutically acceptable salt thereof, or the pharmaceutical composition thereof is administered in one treatment cycle of every 2-6 weeks, e.g., one treatment cycle of every 1 week, every 2 weeks, every 3 weeks, or every 4 weeks. Preferably, every 4 weeks constitutes one treatment cycle, or preferably, every 3 weeks constitutes one treatment cycle.

In some embodiments of the present application, in the method, the therapy, or the use, the content of the compound of formula I or the pharmaceutically acceptable salt thereof in the pharmaceutical composition is a daily dose, and the compound of formula I or the pharmaceutically acceptable salt thereof is administered as follows: administering the compound of formula I or the pharmaceutically acceptable salt thereof once daily.

In some embodiments of the present application, in the method, the therapy, or the use, the content of the compound of formula I or the pharmaceutically acceptable salt thereof in the pharmaceutical composition is a daily dose, wherein the compound of formula I or the pharmaceutically acceptable salt thereof is administered in a single-dose form or in a multi-dose form, typically in a multi-dose form; further, the compound of formula I or the pharmaceutically acceptable salt thereof is administered once daily in a multi-dose form; further, the compound of formula I or the pharmaceutically acceptable salt thereof is consecutively administered once daily in a multi-dose form in each treatment cycle.

In some embodiments of the present application, in the method, the therapy, or the use, the compound of formula I or the pharmaceutically acceptable salt thereof is administered in a daily dose of 100-1200 mg, or in a daily dose of 100-900 mg, or in a daily dose of 200-900 mg, or in a daily dose of 200-800 mg, or in a daily dose of 200-600 mg, or in a daily dose of 400-900 mg, or in a daily dose of 400-600 mg.

In some embodiments of the present application, in the method, the therapy, or the use, the compound of formula I or the pharmaceutically acceptable salt thereof is administered in a daily dose of 400 mg, or in a daily dose of 600 mg, or in a daily dose of 900 mg.

In some embodiments of the present application, in the method, the therapy, or the use, the compound of formula I or the pharmaceutically acceptable salt thereof is administered in a daily dose of 400 mg or in a daily dose of 600 mg.

In some embodiments of the present application, in the method, the therapy, or the use, the compound of formula I or the pharmaceutically acceptable salt thereof is administered in a daily dose of 600 mg. In some embodiments of the present application, in the method, the therapy, or the use, the compound of formula I or the pharmaceutically acceptable salt thereof in the pharmaceutical composition is administered in a multi-dose form consisting of single doses of 50 mg and/or 200 mg of the pharmaceutical composition of the compound of formula I or the pharmaceutically acceptable salt thereof. In some embodiments of the present application, in the method, the therapy, or the use, the compound of formula I or the pharmaceutically acceptable salt thereof is administered based on a regimen of consecutive daily administration.

In some embodiments of the present application, in the method, the therapy, or the use, the compound of formula I or the pharmaceutically acceptable salt thereof in the pharmaceutical composition is administered in a multi-dose form consisting of single doses of 200 mg of the pharmaceutical composition of the compound of formula I or the pharmaceutically acceptable salt thereof. In some embodiments of the present application, in the method, the therapy, or the use, the compound of formula I or the pharmaceutically acceptable salt thereof is administered based on a regimen of consecutive daily administration.

In some embodiments of the present application, in the method, the therapy, or the use, the compound of formula I or the pharmaceutically acceptable salt thereof is administered once daily in a multi-dose form in a daily dose of 400 mg or in a daily dose of 600 mg.

In some embodiments of the present application, in the method, the therapy, or the use, the compound of formula I or the pharmaceutically acceptable salt thereof is administered once daily in a multi-dose form in a daily dose of 600 mg; the daily dose consists of single doses of 200 mg of the pharmaceutical composition of the compound of formula I or the pharmaceutically acceptable salt thereof.

In some embodiments of the present application, in the method, the therapy, or the use, the content of the compound of formula I or the pharmaceutically acceptable salt thereof in the pharmaceutical composition is a dose in each treatment cycle, and the compound of formula I or the pharmaceutically acceptable salt thereof is administered as follows: administering the compound of formula I or the pharmaceutically acceptable salt thereof daily, wherein the compound of formula I or the pharmaceutically acceptable salt thereof is packaged in a single aliquot or in multiple aliquots (e.g., 2, 3, 4, 7, 14, 21, 28, or more aliquots).

In some embodiments of the present application, in the method, the therapy, or the use, 28 days constitutes one treatment cycle, and the total dose of the pharmaceutical composition comprising the compound of formula I or the pharmaceutically acceptable salt thereof administered on days 1-28 in each treatment cycle is 2.8 g-33.6 g. In some embodiments, the total dose of the pharmaceutical composition comprising the compound of formula I or the pharmaceutically acceptable salt thereof is 5.6 g-25.2 g. In some embodiments, the total dose of the pharmaceutical composition comprising the compound of formula I or the pharmaceutically acceptable salt thereof is 5.6 g-16.8 g. In some embodiments, the total dose of the pharmaceutical composition comprising the compound of formula I or the pharmaceutically acceptable salt thereof is 11.2 g-25.2 g. In some embodiments, the total dose of the pharmaceutical composition comprising the compound of formula I or the pharmaceutically acceptable salt thereof is 11.2 g-16.8 g. In some embodiments, the total dose of the pharmaceutical composition comprising the compound of formula I or the pharmaceutically acceptable salt thereof is selected from the group consisting of 11.2 g, 16.8 g, and 25.2 g. In some embodiments, the total dose of the pharmaceutical composition comprising the compound of formula I or the pharmaceutically acceptable salt thereof is selected from the group consisting of 11.2 g and 16.8 g. In some embodiments, the total dose of the pharmaceutical composition comprising the compound of formula I or the pharmaceutically acceptable salt thereof is preferably 16.8 g.

In some embodiments of the present application, in the method, the therapy, or the use, 21 days constitutes one treatment cycle, and the total dose of the pharmaceutical composition comprising the compound of formula I or the pharmaceutically acceptable salt thereof administered on days 1-21 in each treatment cycle is 2.1 g-25.2 g. In some embodiments, the total dose of the pharmaceutical composition comprising the compound of formula I or the pharmaceutically acceptable salt thereof is 4.2 g-18.9 g. In some embodiments, the total dose of the pharmaceutical composition comprising the compound of formula I or the pharmaceutically acceptable salt thereof is 4.2 g-12.6 g. In some embodiments, the total dose of the pharmaceutical composition comprising the compound of formula I or the pharmaceutically acceptable salt thereof is 8.4 g-18.9 g. In some embodiments, the total dose of the pharmaceutical composition comprising the compound of formula I or the pharmaceutically acceptable salt thereof is 8.4 g-12.6 g. In some embodiments, the total dose of the pharmaceutical composition comprising the compound of formula I or the pharmaceutically acceptable salt thereof is selected from the group consisting of 8.4 g, 12.6 g, and 18.9 g. In some embodiments, the total dose of the pharmaceutical composition comprising the compound of formula I or the pharmaceutically acceptable salt thereof is selected from the group consisting of 8.4 g and 12.6 g. In some embodiments, the total dose of the pharmaceutical composition comprising the compound of formula I or the pharmaceutically acceptable salt thereof is preferably 12.6 g.

In some embodiments of the present application, in the method, the therapy, or the use, 28 days constitutes one treatment cycle; the pharmaceutical composition comprising the compound of formula I or the pharmaceutically acceptable salt thereof is administered once daily for 28 consecutive days, and the total dose of the pharmaceutical composition comprising the compound of formula I or the pharmaceutically acceptable salt thereof administered in each treatment cycle is 11.2 g, 16.8 g, or 25.2 g. In some embodiments of the present application, in the method, the therapy, or the use, 21 days constitutes one treatment cycle; the pharmaceutical composition comprising the compound of formula I or the pharmaceutically acceptable salt thereof is administered once daily for 21 consecutive days, and the total dose of the pharmaceutical composition comprising the compound of formula I or the pharmaceutically acceptable salt thereof administered in each treatment cycle is 8.4 g, 12.6 g, or 18.9 g.

In some embodiments of the present application, in the method, the therapy, or the use, 28 days constitutes one treatment cycle; the pharmaceutical composition comprising the compound of formula I or the pharmaceutically acceptable salt thereof is administered once daily for 28 consecutive days, and the total dose of the pharmaceutical composition comprising the compound of formula I or the pharmaceutically acceptable salt thereof administered in each treatment cycle is 11.2 g or 16.8 g. In some embodiments of the present application, in the method, the therapy, or the use, 21 days constitutes one treatment cycle; the pharmaceutical composition comprising the compound of formula I or the pharmaceutically acceptable salt thereof is administered once daily for 21 consecutive days, and the total dose of the pharmaceutical composition comprising the compound of formula I or the pharmaceutically acceptable salt thereof administered in each treatment cycle is 8.4 g or 12.6 g.

In some embodiments of the present application, in the method, the therapy, or the use, 28 days constitutes one treatment cycle; the pharmaceutical composition comprising the compound of formula I or the pharmaceutically acceptable salt thereof is administered once daily for 28 consecutive days, and the total dose of the pharmaceutical composition comprising the compound of formula I or the pharmaceutically acceptable salt thereof administered in each treatment cycle is 16.8 g. In some embodiments of the present application, in the method, the therapy, or the use, 21 days constitutes one treatment cycle; the pharmaceutical composition comprising the compound of formula I or the pharmaceutically acceptable salt thereof is administered once daily for 21 consecutive days, and the total dose of the pharmaceutical composition comprising the compound of formula I or the pharmaceutically acceptable salt thereof administered in each treatment cycle is 12.6 g.

In the embodiments of the present application, the treatment cycle described above is repeated as long as the disease is still under control and the administration regimen is clinically tolerable.

In the embodiments of the present application, the administration is consecutively performed between each treatment cycle, that is, the administration is not stopped after the previous treatment cycle is finished, and the next treatment cycle is consecutively performed.

### Compound of Formula I or Pharmaceutically Acceptable Salt Thereof

In some embodiments of the present application, the compound of formula I described herein may be obtained by reference to a preparation method in WO2017162157 or WO2019057142,

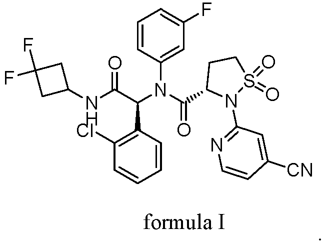

The compound of formula I of the present application may be administered in its free base form, or in the form of its pharmaceutically acceptable salt, hydrate, and prodrug that is converted into the compound of formula I *in vivo.* For example, within the scope of the present application, the pharmaceutically acceptable salt of the compound of formula I may be generated from various organic and inorganic acids according to methods well known in the art. As used herein, the compound of formula I is in a free base thereof.

As used herein, the compound of formula I is in a hydrate form thereof and is administered in its hydrate form, wherein each molecule of the free base contains 0.5-3 water molecules; preferably, each molecule of the free base contains 1 water molecule, as shown in a compound of formula II below:

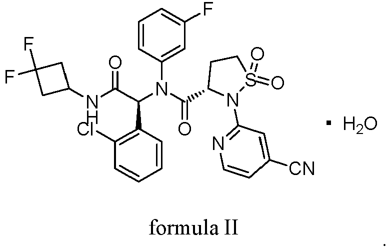

As used herein, the compound of formula II is in a crystalline form.

As used herein, the compound of formula II is in an amorphous solid form.

Unless otherwise stated, the dose of the compound of formula I or the pharmaceutically acceptable salt thereof involved in the present application is based on the molecular weight of the compound of formula I (based on C₂₈H₂₃ClF₃N₃O₄S).

In some embodiments of the present application, the compound of formula II described herein may be obtained by reference to a preparation method in WO2019057142.

As used herein, isomers of the compound of formula I, such as racemates of the compound of formula I, enantiomers of the compound of formula I, tautomers of the compound of formula I, diastereoisomers of the compound of formula I, or mixtures of the isomers described above, are also included within the scope of the present application.

### Pharmaceutical Composition of Compound of Formula I or Pharmaceutically Acceptable Salt Thereof

In some embodiments of the present application, the compound of formula I or the pharmaceutically acceptable salt thereof is a pharmaceutical composition comprising a therapeutically effective amount of the compound of formula I or the pharmaceutically acceptable salt thereof.

In some embodiments of the present application, the pharmaceutical composition comprises the compound of formula I or the pharmaceutically acceptable salt thereof in a single dose of 50 mg or 200 mg by weight of the compound of formula I. Alternatively, the pharmaceutical composition of the compound of formula I or the pharmaceutically acceptable salt thereof is prepared to comprise 50 mg or 200 mg of the compound of formula I or the pharmaceutically acceptable salt thereof by weight of the compound of formula I in a unit formulation.

In some embodiments of the present application, the pharmaceutical composition comprises the compound of formula I or the pharmaceutically acceptable salt thereof in a single dose of 200 mg by weight of the compound of formula I. Alternatively, the pharmaceutical composition of the compound of formula I or the pharmaceutically acceptable salt thereof is prepared to comprise 200 mg of the compound of formula I or the pharmaceutically acceptable salt thereof by weight of the compound of formula I in a unit formulation.

The method of administration can be determined comprehensively depending on the activity and toxicity of the medicament, tolerance of a patient, etc.

In some embodiments of the present application, the pharmaceutical composition includes, but is not limited to formulations suitable for oral, parenteral, and local administrations; in some embodiments, the pharmaceutical composition is a formulation suitable for oral administration; in some embodiments, the pharmaceutical composition is a solid formulation suitable for oral administration; in some embodiments, the pharmaceutical composition includes, but is not limited to a tablet and a capsule.

In some embodiments of the present application, the pharmaceutical composition is a solid pharmaceutical composition.

In some embodiments of the present application, the pharmaceutical composition of the compound of formula I or the pharmaceutically acceptable salt thereof is a solid pharmaceutical composition comprising the compound of formula I or the pharmaceutically acceptable salt thereof.

In some embodiments of the present application, the pharmaceutical composition of the compound of formula I or the pharmaceutically acceptable salt thereof is a tablet comprising the compound of formula I or the pharmaceutically acceptable salt thereof.

In some embodiments of the present application, the pharmaceutical composition of the compound of formula I or the pharmaceutically acceptable salt thereof of the present application may further comprise a pharmaceutically acceptable carrier and/or excipient.

The pharmaceutical composition of the compound of formula I or the pharmaceutically acceptable salt thereof of the present application can be manufactured using methods well known in the art, such as conventional mixing, dissolution, granulation, dragee-making, levigation, emulsifying, lyophilization, etc.

A solid oral composition can be prepared by conventional mixing, filling, or tabletting, for example, the solid oral composition can be obtained by the following method: mixing the active compound with a solid excipient, optionally grinding the resulting mixture, adding additional suitable excipients if desired, and processing the mixture into granules to get the core part of a tablet or dragee. Suitable excipients include, but are not limited to: binders, diluents, disintegrants, lubricants, glidants, sweeteners, flavoring agents, and the like.

### Biliary Tract Cancer

In the present application, the biliary tract cancer comprises primary cancers in the hilar bile duct, the common hepatic duct, and the common bile duct, including cholangiocarcinoma, gallbladder cancer, and ampulla tumor.

In some embodiments of the present application, the biliary tract cancer is biliary tract adenocarcinoma.

In some embodiments of the present application, the biliary tract cancer is biliary cystadenocarcinoma.

In some embodiments of the present application, the biliary tract cancer is cholangiocarcinoma or gallbladder cancer.

In some embodiments of the present application, the biliary tract cancer is intrahepatic cholangiocarcinoma, hilar cholangiocarcinoma, distal cholangiocarcinoma, or gallbladder cancer.

In some embodiments of the present application, the biliary tract cancer is intrahepatic cholangiocarcinoma, extrahepatic cholangiocarcinoma, or gallbladder cancer.

In some embodiments of the present application, the biliary tract cancer is cholangiocarcinoma.

In some embodiments of the present application, the cholangiocarcinoma is intrahepatic cholangiocarcinoma and extrahepatic cholangiocarcinoma.

In some embodiments of the present application, the cholangiocarcinoma is intrahepatic cholangiocarcinoma. In some embodiments of the present application, the intrahepatic cholangiocarcinoma comprises a combined hepatocellular-cholangiocarcinoma component > 50%.

In some embodiments of the present application, the cholangiocarcinoma is cholangiocellular carcinoma.

In some embodiments of the present application, the cholangiocarcinoma is intrahepatic cholangiocellular carcinoma or extrahepatic cholangiocellular carcinoma.

In some embodiments of the present application, the cholangiocarcinoma is intrahepatic cholangiocellular carcinoma.

In some embodiments of the present application, the cholangiocarcinoma is a poorly differentiated cholangiocellular carcinoma, a moderately differentiated cholangiocellular carcinoma, or a well-differentiated cholangiocellular carcinoma.

In some embodiments of the present application, the cholangiocarcinoma is a poorly differentiated cholangiocellular carcinoma.

In some embodiments of the present application, the cholangiocarcinoma is a moderately differentiated cholangiocellular carcinoma.

In some embodiments of the present application, the cholangiocarcinoma is a poorly differentiated intrahepatic cholangiocellular carcinoma or a moderately differentiated intrahepatic cholangiocellular carcinoma.

In some embodiments of the present application, the cholangiocarcinoma is a poorly differentiated extrahepatic cholangiocellular carcinoma or a moderately differentiated extrahepatic cholangiocellular carcinoma.

In some embodiments of the present application, the biliary tract cancer is biliary tract cancer with an IDH1 gene mutation.

In some embodiments of the present application, the IDH1 gene mutation is an IDH1 R132 gene mutation.

In some embodiments of the present application, the IDH1 gene mutation includes, but is not limited to, one or more of R132C, R132G, R132H, R132L, or R132S mutation.

In some embodiments of the present application, the biliary tract cancer is advanced biliary tract cancer.

In some embodiments of the present application, the biliary tract cancer is advanced biliary tract cancer with an IDH1 mutation.

In some embodiments of the present application, the biliary tract cancer is locally advanced, recurrent, and/or metastatic biliary tract cancer.

In some embodiments of the present application, the biliary tract cancer is locally advanced, recurrent, and/or metastatic biliary tract cancer with an IDH1 mutation.

In some embodiments of the present application, the biliary tract cancer is locally advanced, recurrent, and/or metastatic biliary tract cancer with an IDH1 mutation, and the biliary tract cancer is intrahepatic cholangiocarcinoma, hilar cholangiocarcinoma, distal cholangiocarcinoma, or gallbladder cancer. Preferably, the biliary tract cancer is intrahepatic cholangiocarcinoma.

In some embodiments of the present application, the biliary tract cancer is unresectable advanced biliary tract cancer. In some embodiments of the present application, the biliary tract cancer is unresectable locally advanced, recurrent, and/or metastatic biliary tract cancer.

In some embodiments of the present application, the biliary tract cancer is biliary tract cancer having at least 1 measurable lesion according to RECIST 1.1 criteria.

In some embodiments of the present application, the biliary tract cancer is unresectable locally advanced, recurrent, and/or metastatic biliary tract cancer having at least 1 measurable lesion according to RECIST 1.1 criteria.

In some embodiments of the present application, the biliary tract cancer is locally advanced, recurrent, and/or metastatic biliary tract cancer that has not previously been treated systemically for advanced disease.

In some embodiments of the present application, the biliary tract cancer is unresectable locally advanced, recurrent, and/or metastatic biliary tract cancer that has not previously been treated systemically for advanced disease. In some embodiments of the present application, the biliary tract cancer is unresectable locally advanced, recurrent, and/or metastatic biliary tract cancer having at least 1 measurable lesion according to RECIST 1.1 criteria that has not previously been treated systemically for advanced disease.

In some embodiments of the present application, the biliary tract cancer is biliary tract cancer that has previously failed treatment with a gemcitabine, and/or fluorouracil, and/or platinum-based drug. In some embodiments of the present application, the biliary tract cancer is unresectable locally advanced, recurrent, and/or metastatic biliary tract cancer that has previously failed treatment with a gemcitabine, and/or fluorouracil, and/or platinum-based drug. In some embodiments of the present application, the biliary tract cancer is unresectable locally advanced, recurrent, and/or metastatic biliary tract cancer that has previously failed treatment with a gemcitabine and/or fluorouracil drug.

In some embodiments of the present application, the biliary tract cancer is locally advanced, recurrent, and/or metastatic biliary tract cancer with an IDH1 mutation that has previously failed treatment with a gemcitabine, and/or fluorouracil, and/or platinum-based drug.

In some embodiments of the present application, the biliary tract cancer is locally advanced, recurrent, and/or metastatic biliary tract cancer that has previously failed first-line and second-line treatments.

In some embodiments of the present application, the biliary tract cancer is unresectable locally advanced, recurrent, and/or metastatic biliary tract cancer that has previously failed first-line and second-line treatments. In some embodiments of the present application, the biliary tract cancer is unresectable locally advanced, recurrent, and/or metastatic biliary tract cancer having at least 1 measurable lesion according to RECIST 1.1 criteria that has previously failed first-line and second-line treatments.

In some embodiments of the present application, the first-line and second-line treatments comprise one or more of surgical treatment, radiotherapy, and drug therapy.

In some embodiments of the present application, the drug therapy comprises one or more of chemotherapy, targeted drug therapy, or immune checkpoint inhibitor therapy.

In some embodiments of the present application, the chemotherapy comprises one or more of gemcitabine, capecitabine, fluorouracil, tegafur-gimeracil-oteracil potassium, platinums, taxanes, vinca alkaloids, adriamycin, epirubicin, mitoxantrone, irinotecan, docetaxel, pemetrexed, raltitrexed, methotrexate, or temozolomide.

In some embodiments of the present application, the chemotherapy comprises one or more of gemcitabine, capecitabine, epirubicin, fluorouracil, tegafur-gimeracil-oteracil potassium, platinums, and taxanes.

In some embodiments of the present application, the platinums comprise cisplatin, carboplatin, nedaplatin, lobaplatin, and oxaliplatin, preferably cisplatin and oxaliplatin.

In some embodiments of the present application, the taxanes comprise paclitaxel, docetaxel, liposomal paclitaxel, and albumin-bound paclitaxel, preferably albumin-bound paclitaxel.

In some embodiments of the present application, the vinca alkaloids comprise vinblastine, vincristine, vinorelbine, or vindesine.

In some embodiments of the present application, the chemotherapy comprises a combination treatment of gemcitabine and cisplatin; or a combination treatment of gemcitabine and oxaliplatin; or a combination treatment of gemcitabine and fluorouracil; or a combination treatment of gemcitabine and tegafur-gimeracil-oteracil potassium.

In some embodiments of the present application, the targeted drug therapy comprises one or more of lenvatinib, apatinib, anlotinib, gefitinib, erlotinib, afatinib, imatinib, nilotinib, sunitinib, lapatinib, bosutinib, vandetanib, sunitinib, erlotinib, neratinib, dasatinib, canertinib, canertinib, dasatinib, saracatinib, sunitinib, olaparib, niraparib, bevacizumab, or recombinant human endostatin.

In some embodiments of the present application, the targeted drug therapy comprises one or more of lenvatinib, apatinib, olaparib, niraparib, bevacizumab, or recombinant human endostatin.

In some embodiments of the present application, the immune checkpoint inhibitor comprises a PD-1 inhibitor and/or a PD-L1 inhibitor, and the PD-1 inhibitor and/or the PD-L1 inhibitor comprises one or more of pembrolizumab, nivolumab, atezolizumab, avelumab, durvalumab, cemiplimab, dostarlimab, camrelizumab, tislelizumab, sintilimab, toripalimab, or penpulimab.

In some embodiments of the present application, the immune checkpoint inhibitor comprises one or more of pembrolizumab, durvalumab, toripalimab, tislelizumab, sintilimab, or camrelizumab.

In some embodiments of the present application, the drug therapy comprises a single-drug therapy or a combination treatment of multiple drugs.

In some embodiments of the present application, the single-drug therapy comprises lenvatinib, apatinib, olaparib, niraparib, pembrolizumab, durvalumab, gemcitabine, oxaliplatin, fluorouracil, sintilimab, or camrelizumab.

In some embodiments of the present application, the combination treatment of multiple drugs comprises a combination treatment of chemotherapy and an immune checkpoint inhibitor; or a combination treatment of a targeted drug and an immune checkpoint inhibitor; or a combination treatment of chemotherapy and a targeted drug; or a combination treatment of chemotherapy, a targeted drug, and an immune checkpoint inhibitor.

In some embodiments of the present application, the combination treatment of multiple drugs comprises a combination treatment of gemcitabine, cisplatin, and an immune checkpoint inhibitor; or a combination treatment of gemcitabine, tegafur-gimeracil-oteracil potassium, and an immune checkpoint inhibitor; or a combination treatment of gemcitabine, oxaliplatin, and an immune checkpoint inhibitor; or a combination treatment of lenvatinib, cisplatin, and an immune checkpoint inhibitor; or a combination treatment of gemcitabine, cisplatin, lenvatinib, and an immune checkpoint inhibitor.

In some embodiments of the present application, the combination treatment of multiple drugs comprises a combination treatment of toripalimab, bevacizumab, oxaliplatin, and fluorouracil; or a combination treatment of recombinant human endostatin and cisplatin; or a combination treatment of gemcitabine, cisplatin, and tislelizumab; or a combination treatment of gemcitabine and tegafur-gimeracil-oteracil potassium; or a combination treatment of gemcitabine and cisplatin; or a combination treatment of capecitabine, and lenvatinib; or a combination treatment of lenvatinib, cisplatin, and pembrolizumab; or a combination treatment of lenvatinib, gemcitabine, cisplatin, and pembrolizumab; or a combination treatment of gemcitabine and oxaliplatin; or a combination treatment of gemcitabine and sintilimab; or a combination treatment of apatinib and camrelizumab; or a combination treatment of lenvatinib and camrelizumab; or a combination treatment of gemcitabine, oxaliplatin, and sintilimab; or a combination treatment of gemcitabine and albumin-bound paclitaxel; or a combination treatment of gemcitabine and tegafur-gimeracil-oteracil potassium; or a combination treatment of capecitabine and sintilimab; or a combination treatment of capecitabine, oxaliplatin, and sintilimab; or a combination treatment of gemcitabine, oxaliplatin, and albumin-bound paclitaxel; or a combination treatment of camrelizumab, gemcitabine, oxaliplatin, and albumin-bound paclitaxel; or a combination treatment of lenvatinib and niraparib; or a combination treatment of gemcitabine, cisplatin, anlotinib, and sintilimab.

In some embodiments of the present application, the failure of the drug therapy or the failure of the first-line and second-line treatments means that the administration time of the previous therapy is ≥ 1 treatment cycle, and the disease progression meeting the definition of RECIST 1.1 criteria occurs during the treatment cycle or within 6 months after the end of the treatment, or refers to intolerance to chemotherapy toxicity; or the failure of the drug therapy or the failure of the first-line and second-line treatments means that neoadjuvant or adjuvant therapy has been previously given, and the disease progression or disease recurrence meeting the definition of RECIST 1.1 criteria occurs during the treatment or within 6 months after the end of the treatment; or the failure of the drug therapy or the failure of the first-line and second-line treatments refers to the disease progression of intrahepatic lesions meeting the definition of RECIST 1.1 criteria during treatment with hepatic arterial infusion chemotherapy or within 6 months after the last treatment.

In some embodiments of the present application, the neoadjuvant or adjuvant therapy is adjuvant radiotherapy or chemotherapy after surgical resection of biliary tract cancer, and the chemotherapy is as described above.

In some embodiments of the present application, the cholangiocarcinoma is advanced cholangiocarcinoma.

In some embodiments of the present application, the cholangiocarcinoma is advanced cholangiocarcinoma with an IDH1 gene mutation.

In some embodiments of the present application, the cholangiocarcinoma is locally advanced, recurrent, and/or metastatic cholangiocarcinoma.

In some embodiments of the present application, the cholangiocarcinoma is locally advanced, recurrent, and/or metastatic cholangiocarcinoma with an IDH1 mutation.

In some embodiments of the present application, the cholangiocarcinoma is locally advanced, recurrent, and/or metastatic cholangiocarcinoma with an IDH1 mutation, and the cholangiocarcinoma is intrahepatic cholangiocarcinoma, hilar cholangiocarcinoma, or distal cholangiocarcinoma. Preferably, the cholangiocarcinoma is intrahepatic cholangiocarcinoma.

In some embodiments of the present application, the cholangiocarcinoma is unresectable advanced cholangiocarcinoma.

In some embodiments of the present application, the cholangiocarcinoma is unresectable locally advanced, recurrent, and/or metastatic cholangiocarcinoma.

In some embodiments of the present application, the cholangiocarcinoma is cholangiocarcinoma having at least 1 measurable lesion according to RECIST 1.1 criteria.

In some embodiments of the present application, the cholangiocarcinoma is unresectable locally advanced, recurrent, and/or metastatic cholangiocarcinoma having at least 1 measurable lesion according to RECIST 1.1 criteria.

In some embodiments of the present application, the cholangiocarcinoma is locally advanced, recurrent, and/or metastatic cholangiocarcinoma that has not previously been treated systemically for advanced disease.

In some embodiments of the present application, the cholangiocarcinoma is unresectable locally advanced, recurrent, and/or metastatic cholangiocarcinoma that has not previously been treated systemically for advanced disease. In some embodiments of the present application, the cholangiocarcinoma is unresectable locally advanced, recurrent, and/or metastatic cholangiocarcinoma having at least 1 measurable lesion according to RECIST 1.1 criteria that has not previously been treated systemically for advanced disease.

In some embodiments of the present application, the cholangiocarcinoma is cholangiocarcinoma that has previously failed treatment with a gemcitabine, and/or fluorouracil, and/or platinum-based drug. In some embodiments of the present application, the cholangiocarcinoma is unresectable locally advanced, recurrent, and/or metastatic cholangiocarcinoma that has previously failed treatment with a gemcitabine, and/or fluorouracil, and/or platinum-based drug. In some embodiments of the present application, the cholangiocarcinoma is unresectable locally advanced, recurrent, and/or metastatic cholangiocarcinoma that has previously failed treatment with a gemcitabine and/or fluorouracil drug.

In some embodiments of the present application, the cholangiocarcinoma is locally advanced, recurrent, and/or metastatic cholangiocarcinoma that has previously failed first-line and second-line treatments.

In some embodiments of the present application, the cholangiocarcinoma is unresectable locally advanced, recurrent, and/or metastatic cholangiocarcinoma that has previously failed first-line and second-line treatments. In some embodiments of the present application, the cholangiocarcinoma is unresectable locally advanced, recurrent, and/or metastatic cholangiocarcinoma having at least 1 measurable lesion according to RECIST 1.1 criteria that has previously failed first-line and second-line treatments.

In some embodiments of the present application, the cholangiocarcinoma is unresectable locally advanced, recurrent, and/or metastatic intrahepatic cholangiocarcinoma that has previously failed first-line and second-line treatments.

In some embodiments of the present application, the cholangiocarcinoma is unresectable locally advanced, recurrent, and/or metastatic hilar cholangiocarcinoma that has previously failed first-line and second-line treatments.

In some embodiments of the present application, the cholangiocarcinoma is unresectable locally advanced, recurrent, and/or metastatic distal cholangiocarcinoma that has previously failed first-line and second-line treatments.

In some embodiments of the present application, the cholangiocarcinoma is unresectable locally advanced, recurrent, and/or metastatic cholangiocellular carcinoma that has previously failed first-line and second-line treatments.

In some embodiments of the present application, the cholangiocarcinoma is unresectable locally advanced, recurrent, and/or metastatic intrahepatic cholangiocellular carcinoma that has previously failed first-line and second-line treatments.

In some embodiments of the present application, the cholangiocarcinoma is an unresectable locally advanced, recurrent, and/or metastatic poorly differentiated intrahepatic cholangiocellular carcinoma that has previously failed first-line and second-line treatments.

In some embodiments of the present application, the cholangiocarcinoma is an unresectable locally advanced, recurrent, and/or metastatic moderately differentiated intrahepatic cholangiocellular carcinoma that has previously failed first-line and second-line treatments.

In some embodiments of the present application, the biliary tract cancer or cholangiocarcinoma may coexist with liver cancer.

### Mode of administration

The content below is not intended to limit the mode of administration of the pharmaceutical composition of the present application.

The components in the pharmaceutical composition of the present application can be administered in various proper routes including, but not limited to, oral administration or parenteral administration (by intravenous, intramuscular, local, or subcutaneous routes). In some embodiments, the components of the pharmaceutical composition of the present application can be administered via oral administration or injection, for example, intravenous injection or subcutaneous injection.

Suitable dosage forms of the pharmaceutical composition of the present application include, but are not limited to, tablets, lozenges, pills, capsules (e.g., hard capsules, soft capsules, enteric capsules, and microcapsules), elixirs, granules, syrups, injections (intramuscular, intravenous, and intraperitoneal), granules, emulsions, suspensions, solutions, dispersants, and dosage forms of sustained-release formulations for oral or non-oral administration.

### Technical effects

The compound of formula I or the pharmaceutically acceptable salt thereof of the present application has good safety and anti-tumor activity, and patients have good tolerance during the treatment. Moreover, the compound of formula I or the pharmaceutically acceptable salt thereof can provide relatively good disease control rate (DCR) and objective response rate (ORR), relatively long duration of response (DOR), and relatively long survival (e.g., median survival, progression-free survival, or overall survival) in the treated patients, and thus can exhibit good clinical benefits.

### Definitions and description

Unless otherwise stated, the terms used herein shall have the following meanings. A certain term, unless otherwise specifically defined, should not be considered uncertain or unclear, but construed according to its common meaning in the art. When referring to a trade name herein, it is intended to refer to its corresponding commercial product or its active ingredient.

As used herein, unless otherwise stated, the terms "comprise", "comprises", and "comprising" or equivalents thereof are open-ended statements and mean that elements, components, and steps that are not specified may be encompassed in addition to those listed.

All patents, patent applications, and other identified publications are explicitly incorporated herein by reference for the purpose of description and disclosure. Any reference to these publications herein shall not be construed as an admission that the publications form part of the commonly recognized knowledge in the art.

The term "pharmaceutically acceptable" is used for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids or salts formed from acidic radicals and free bases.

As used herein, the content of the compound of formula I or the pharmaceutically acceptable salt thereof, e.g., the amount administered, the dose, or the content in the pharmaceutical composition, is calculated based on the free base form.

As used herein, if the compound in the pharmaceutical combination of the present application has, for example, at least one basic site, an acid addition salt may be formed. If needed, the compound may further form a corresponding acid addition salt with additionally present basic sites. A compound with at least one acidic group (e.g., COOH) may further form a salt with a base. If the compound comprises, for example, both carboxyl and amino, the compound may further form a corresponding inner salt.

The terms "patient", "subject", and "individual" are used interchangeably and refer to mammals such as humans, dogs, cows, horses, pigs, sheep, goats, cats, mice, rabbits, rats, and transgenic non-human animals. In some embodiments, the patient, subject, or individual is a human.

The term "pharmaceutical composition" refers to a mixture comprising one or more of the compounds or the salts thereof of the present application and a pharmaceutically acceptable excipient. The pharmaceutical composition is intended to facilitate the administration of the compound or the salt thereof of the present application to a patient. The pharmaceutical composition of the present application may be administered to a subject in combination with additional drugs/therapeutic agents and the like.

The term "combination" does not mean that two or more different subjects to which it refers are necessarily administered simultaneously, but rather that they are to be understood as being administered separately, either sequentially, simultaneously, or at different frequencies of administration.

The term "treat", "treating", or "treatment" generally refers to obtaining desired pharmacological and/or physiological effects, including partially or completely stabilizing or curing a disease and/or an effect caused by the disease. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application used for (i) treating a specific disease, condition, or disorder, or (ii) relieving, ameliorating, or eliminating one or more symptoms of a specific disease, condition, or disorder. The amount of the compound of the present application that is considered as the "therapeutically effective amount" varies depending on the compound, the disease state and its severity, the mode of administration, and the age of the mammal to be treated, but can be determined routinely by those skilled in the art in accordance with their knowledge and the present disclosure.

The term "single dose" refers to the smallest unit of packaging containing a certain quantity of pharmaceutical product, for example, in a box of seven capsules, each capsule is a single dose; or a vial of injection can be a single dose.

Unless otherwise specified clearly in the context, singular terms herein encompass plural referents, and vice versa. Similarly, unless otherwise specified clearly in the context, the word "or" herein is intended to include "and". Those skilled in the art will recognize that the scope of the present application is not limited to the embodiments described above. Instead, various modifications, substitutions, or recombinations may be made without departing from the spirit of the present application, and the embodiments resulting from these adjustments all fall within the protection scope of the present application.

### BRIEF DESCRIPTION OF THE DRAWINGS

FIG. 1 shows the relationships between exposure AUC₍₀₋₂₄₎ and dose in patients after a single administration (D1) and after administration for 28 consecutive days (D28) in Experimental Example 1.
FIG. 2 shows the relationships of the maximum plasma concentration (Cmax) and average steady-state plasma concentration (Cavg) to the dose in patients after a single administration (D1) and after administration for 28 consecutive days (D28) in Experimental Example 1.

### DETAILED DESCRIPTION

The present disclosure is illustrated in more detail through specific examples. The following examples are provided for illustrative purposes only and are not intended to limit the present disclosure in any way.

### Example 1 Clinical Trial of Biliary Tract Cancer

Patients with locally advanced, recurrent, and/or metastatic biliary tract cancer, who had previously failed ≥ first-line chemotherapy with a combination regimen based on gemcitabine or fluorouracil, were enrolled in this study. All study drugs were tablets of the compound of formula I, and the purpose of this study was to evaluate the safety and efficacy of the treatment with the compound of formula I.

### 1.1 Test drug and administration regimen

### 1.1.1 Test drug:

Tablets of the compound of formula I, with the strength of 50 mg/tablet and/or 200 mg/tablet, provided by Chia Tai Tianqing Pharmaceutical Group Co., Ltd.

### 1.1.2 Administration regimen:

Tablets of the compound of formula I: a daily dose of 400 mg, 600 mg, or 900 mg, oral administration at fasting, once daily, and 28 consecutive days of administration as one treatment cycle.

### 1.2 Enrollment criteria

1) 18 years ≤ age ≤ 75 years (based on the date of signing the informed consent); ECOG score: 0-1; expected survival ≥ 12 weeks.
2) An IDH1 R132 gene mutation (one of R132C, R132G, R132H, R132L, and R132S) was present.
3) With good main organ functions meeting the following criteria:
   a) Blood biochemical examination results should meet the following criteria:
      i. Alanine aminotransferase (ALT) and aspartate aminotransferase (AST) ≤ 3 × ULN.
         If liver metastasis is accompanied, ALT and AST ≤ 5 × ULN;
      ii. Serum creatinine (Cr) ≤ 1.5 × ULN, or creatinine clearance rate as estimated by Cockcroft-Gault glomerular filtration equation ≥ 50 mL/min.
   b) Urine routine examination criteria: the urine routine indicates that urine protein < ++; if the urine protein ≥ ++, the quantitative determination of the urine protein in 24 hours needs to be confirmed to ≤ 1.0 g.
   c) Echocardiography evaluation: left ventricular ejection fraction (LVEF) ≥ 50%.
   d) 12-lead electrocardiogram evaluation: QTc < 450 ms (male) and QTc < 470 ms (female).
4) Female subjects of childbearing age should agree to take effective contraceptive measures during the study and for 6 months after the study; serum or urine pregnancy test results should be negative within 7 days before enrollment; male subjects should agree to take effective contraceptive measures during the study and for 6 months after the study.
5) Subjects with voluntary participation in this study, signed the informed consent, and good compliance.
6) Criteria for advanced biliary tract cancer:
   a) Histologically or cytologically confirmed biliary tract adenocarcinoma, including intrahepatic cholangiocarcinoma (ICC), extrahepatic cholangiocarcinoma (ECC), and gallbladder cancer (GBC).
   b) Unresectable locally advanced, recurrent, and/or metastatic diseases having at least 1 measurable lesion according to RECIST 1.1 criteria.
   c) Failure of at least one chemotherapy with a combination regimen based on gemcitabine or fluorouracil, meeting any one of the following: i) the administration time of the previous systemic treatment for advanced disease was ≥ 1 treatment cycle, and the disease progression meeting the definition of RECIST 1.1 criteria occurred during the last-line treatment or within 6 months after the end of the treatment, or intolerance to chemotherapy toxicity occurred; ii) neoadjuvant or adjuvant therapy had been previously given, and the disease progression or disease recurrence meeting the definition of RECIST 1.1 criteria occurred during the treatment or within 6 months after the end of the treatment.
   d) Criteria for blood routine examination (no blood transfusion and no correction with hematopoietic stimulating drugs within 7 days before the screening): absolute neutrophil count (ANC) ≥ 1.5 × 10⁹/L; platelet ≥ 100 × 10⁹/L; hemoglobin ≥ 9 g/dL.
   e) In the blood biochemistry, total bilirubin (TBIL) ≤ 2 × upper limit of normal value (ULN) (for patients with Gilbert syndrome, ≤ 3 × ULN); serum albumin ≥ 28 g/L.
   f) Criteria for blood coagulation examination: prothrombin time (PT), activated partial thromboplastin time (APTT), and international normalized ratio (INR) ≤ 1.5 × ULN (not previously received anticoagulant therapy).
   g) Body weight ≥ 40 kg and BMI ≥ 18.5 kg/m².

### 1.3 Evaluation method and measure

Safety evaluation criteria: the severity of adverse events was determined using the NCI-CTC AE 5.0 criteria. Efficacy evaluation criteria: RECIST 1.1 criteria were used for evaluation.

### 1.4 Test results

### 1.4.1 Safety

The tablets of the compound of formula I exhibited good tolerance and were safe and controllable. The incidence of AEs (adverse events) in each dose group was relatively low, with the lowest overall incidence of AEs in the 600 mg dose group.

### 1.4.2 Efficacy

A total of 34 patients could be evaluated. Among them, 3 patients were in partial response (PR), 19 patients exhibited stable disease (SD), 12 patients were in disease progression (PD), ORR was 9% (3/34), and DCR was 65% (22/34). Therefore, the results show that the compound of formula I of the present application has clinical benefits for treating biliary tract cancer with an IDH1 gene mutation. Table 1 shows the efficacy data of representative patients.

**Table 1. Efficacy data of representative patients**

| Subject No. | Clinical diagnosis | Dose group (mg/qd) | Administration cycle | IDH1 mutation | Best efficacy | Efficacy evaluation-tumor shrinkage rate (%) | | | | | | |
|---|---|---|---|---|---|---|---|---|---|---|---|---|
| | | | | | | C2 | C4 | C6 | C8 | C10 | C12 | C14 |
| 1 | Postoperative liver metastasis of cholangiocellular carcinoma | 600 | C14 | IDH1-R132C | PR | -22.77 | -30.69 | -30.69 | -30.69 | -30.69 | -30.69 | -17.82 |
| 2 | Intrahepatic cholangiocarcinoma | 400 | C8 | IDH1-R132C | PR | -5.99 | -25.55 | -31.55 | -3.79 | | | |
| 3 | Cholangiocellular carcinoma | 900 | C14 | IDH1-R132 | SD | -20.14 | -25.00 | -28.47 | -27.08 | -27.08 | -27.08 | -26.39 |
| 4 | Poorly differentiated cholangiocellular carcinoma | 600 | C6 | IDH1-R132G | SD | -13.40 | -20.75 | -20.75 | | | | |
| 5 | Cholangiocarcinoma | 600 | C8 | IDH1-R132C | PR | -5.20 | -39.72 | -50.59 | -47.80 | | | |

| | | | | | | | | | | | | |
|---|---|---|---|---|---|---|---|---|---|---|---|---|
| Note: C represents a 28-day treatment cycle, and Cn represents n treatment cycles administered to the patient (i.e., the patient was given the drug for n × 28 days). | | | | | | | | | | | | |

### 1.4.3 PK study

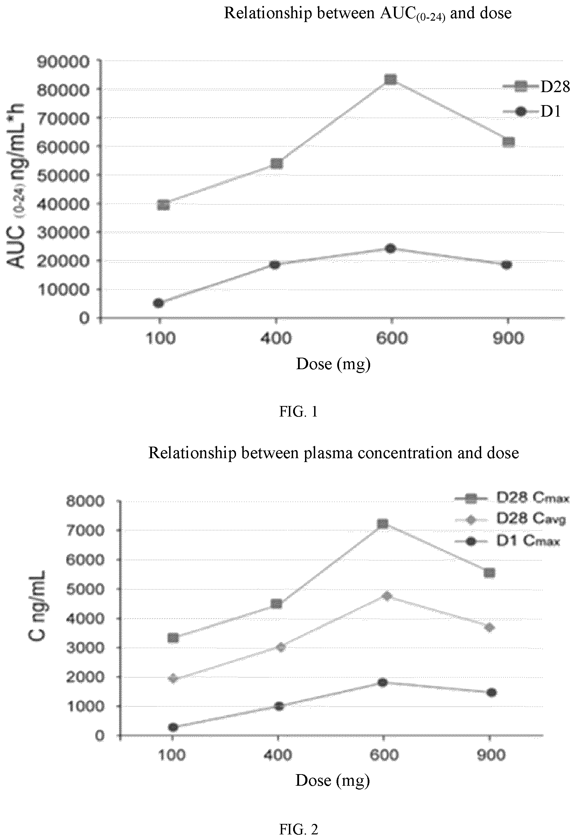

For each dose group, the exposure AUC₍₀₋₂₄₎, maximum plasma concentration (Cmax), and average steady-state plasma concentration (Cavg) in the patients were measured after a single administration (D1) and after administration for 28 consecutive days (D28). The results are shown in FIG. 1 and FIG. 2.

The results show that the 600 mg dose group reached maximum values in exposure AUC₍₀₋₂₄₎, maximum plasma concentration (Cmax), and average steady-state plasma concentration (Cavg).

### Example 2 Clinical Trial of Biliary Tract Cancer

Enrollment in this study: patients with locally advanced, recurrent, and/or metastatic biliary tract cancer, who had not previously received systemic treatment for advanced disease. All study drugs were tablets of the compound of formula I in combination with chemotherapeutic drugs, and the purpose of this study was to evaluate the safety and efficacy of the treatment with the compound of formula I in combination with chemotherapeutic drugs.

### 2.1 Test drug and administration regimen

### 2.1.1 Test drugs:

Tablets of the compound of formula I, with the strength of 50 mg/tablet and/or 200 mg/tablet, provided by Chia Tai Tianqing Pharmaceutical Group Co., Ltd;
chemotherapeutic drugs: CapOX regimen (a systemic treatment regimen recommended in CSCO Guidelines for Diagnosis and Treatment of Biliary Tract Malignancies 2022).

### 2.1.2 Administration regimen:

Tablets of the compound of formula I: a daily dose of 400 mg or 600 mg, oral administration at fasting, once daily, and 21 or 28 consecutive days of administration as one treatment cycle.

### 2.2 Enrollment criteria

1) 18 years ≤ age ≤ 75 years (based on the date of signing the informed consent); ECOG score: 0-1; expected survival ≥ 12 weeks.
2) An IDH1 R132 gene mutation (one of R132C, R132G, R132H, R132L, and R132S) was present.
3) With good main organ functions meeting the following criteria:
   a) Blood biochemical examination results should meet the following criteria:
      i. Alanine aminotransferase (ALT) and aspartate aminotransferase (AST) ≤ 3 × ULN.
         If liver metastasis is accompanied, ALT and AST ≤ 5 × ULN;
      ii. Serum creatinine (Cr) ≤ 1.5 × ULN, or creatinine clearance rate as estimated by Cockcroft-Gault glomerular filtration equation ≥ 50 mL/min.
   b) Urine routine examination criteria: the urine routine indicates that urine protein < ++; if the urine protein ≥ ++, the quantitative determination of the urine protein in 24 hours needs to be confirmed to ≤ 1.0 g.
   c) Echocardiography evaluation: left ventricular ejection fraction (LVEF) ≥ 50%.
   d) 12-lead electrocardiogram evaluation: QTc < 450 ms (male) and QTc < 470 ms (female).
4) Female subjects of childbearing age should agree to take effective contraceptive measures during the study and for 6 months after the study; serum or urine pregnancy test results should be negative within 7 days before enrollment; male subjects should agree to take effective contraceptive measures during the study and for 6 months after the study.
5) Subjects with voluntary participation, signed the informed consent, and good compliance.
6) Criteria for advanced biliary tract cancer:
   a) Histologically or cytologically confirmed biliary tract adenocarcinoma, including intrahepatic cholangiocarcinoma (ICC), extrahepatic cholangiocarcinoma (ECC), and gallbladder cancer (GBC).
   b) Unresectable locally advanced, recurrent, and/or metastatic diseases having at least 1 measurable lesion according to RECIST 1.1 criteria.
   c) Patients who had not previously received systemic treatment for advanced disease may be acceptable in the following two cases: i) patients who had previously received neoadjuvant or adjuvant therapy and exhibited disease progression/recurrence 12 months after the end of the treatment; ii) patients who had previously received systemic treatment for less than 1 cycle and refused the treatment due to their own will were eligible, but patients who stopped the treatment due to toxicity intolerance were excluded.
   d) Criteria for blood routine examination (no blood transfusion and no correction with hematopoietic stimulating drugs within 7 days before the screening): absolute neutrophil count (ANC) ≥ 1.5 × 10⁹/L; platelet ≥ 100 × 10⁹/L; hemoglobin ≥ 9 g/dL.
   e) In the blood biochemistry, total bilirubin (TBIL) ≤ 2 × upper limit of normal value (ULN) (for patients with Gilbert syndrome, ≤ 3 × ULN); serum albumin ≥ 28 g/L.
   f) Criteria for blood coagulation examination: prothrombin time (PT), activated partial thromboplastin time (APTT), and international normalized ratio (INR) ≤ 1.5 × ULN (not previously received anticoagulant therapy).
   g) Body weight ≥ 40 kg and BMI ≥ 18.5 kg/m².

### 2.3 Evaluation method and measure

Safety evaluation criteria: the severity of adverse events was determined using the NCI-CTC AE 5.0 criteria. Efficacy evaluation criteria: RECIST 1.1 criteria were used for evaluation.

### 2.4 Test results

### 2.4.1 Safety

The tablets of the compound of formula I in combination with chemotherapeutic drugs exhibited good tolerance and were safe and controllable. The incidence of AEs in each dosage group was relatively low.

### 2.4.2 Efficacy

A total of 5 patients could be evaluated. Among them, 1 patient was in partial response (PR), 3 patients exhibited stable disease (SD), 1 patient was in disease progression (PD), ORR was 20% (1/5), and DCR was 80% (4/5). Therefore, the results show that the compound of formula I of the present application in combination with chemotherapeutic drugs has clinical benefits for treating biliary tract cancer with an IDH1 gene mutation. Table 2 shows the efficacy data of a representative patient.

**Table 2. Efficacy data of a representative patient**

| Subject No. | Clinical diagnosis | Dose group | Administration cycle | IDH1 mutation | Best efficacy | Efficacy evaluation-tumor shrinkage rate (%) | | | | | |
|---|---|---|---|---|---|---|---|---|---|---|---|
| | | | | | | C2 | C4 | C6 | C8 | C10 | C12 |
| 1 | Intrahepatic cholangiocarcinoma | Tablet of compound of formula I, 600 mg/qd; in combination with the CapOX regimen; | C12 | IDH1-R132L | PR | -25.00 | -25.00 | -25.00 | -30.00 | -30.00 | -30.00 |
| | | 21 days as 1 treatment cycle | | | | | | | | | |

| | | | | | | | | | | | |
|---|---|---|---|---|---|---|---|---|---|---|---|
| Note: The CapOX regimen includes: oxaliplatin for injection was intravenously infused at 130 mg/m² on the 1st day of each treatment cycle, and the capecitabine tablet was orally taken at a total daily dose of 2000 mg/m² 2 times, in the morning and evening, half an hour after each meal, with consecutively 2-week administration and then 1-week interruption. | | | | | | | | | | | |

## Claims

1. A compound of formula I or a pharmaceutically acceptable salt thereof for use in treating biliary tract cancer in a patient,

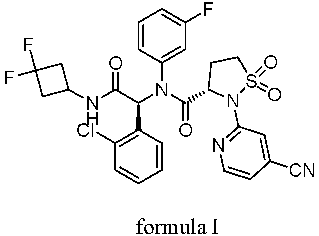

2. A pharmaceutical composition for use in treating biliary tract cancer in a patient, comprising the compound of formula I or the pharmaceutically acceptable salt thereof according to claim 1.

3. The pharmaceutical composition for use according to claim 2, wherein the pharmaceutical composition comprises 100-1200 mg of the compound of formula I or the pharmaceutically acceptable salt thereof by weight of the compound of formula I; preferably, the pharmaceutical composition comprises 100-900 mg, 200-900 mg, 200-800 mg, 200-600 mg, 400-900 mg, 400-600 mg, 400 mg, 600 mg, or 900 mg of the compound of formula I or the pharmaceutically acceptable salt thereof by weight of the compound of formula I.

4. The pharmaceutical composition for use according to claim 2 or 3, wherein the pharmaceutical composition is in a single-dose form or in a multi-dose form; preferably, the pharmaceutical composition is in a multi-dose form.

5. The compound of formula I or the pharmaceutically acceptable salt thereof, or the pharmaceutical composition for use according to any one of claims 1-4, wherein the compound of formula I or the pharmaceutically acceptable salt thereof, or the pharmaceutical composition thereof is administered in one treatment cycle of every 2-6 weeks, every 2 weeks, every 3 weeks, or every 4 weeks.

6. The pharmaceutical composition for use according to any one of claims 2-5, wherein the content of the compound of formula I or the pharmaceutically acceptable salt thereof in the pharmaceutical composition is a daily dose, and the compound of formula I or the pharmaceutically acceptable salt thereof is administered as follows: administering the compound of formula I or the pharmaceutically acceptable salt thereof once daily.

7. The compound of formula I or the pharmaceutically acceptable salt thereof, or the pharmaceutical composition for use according to any one of claims 1-6, wherein the compound of formula I or the pharmaceutically acceptable salt thereof is administered in a daily dose of 100-1200 mg, or in a daily dose of 100-900 mg, or in a daily dose of 200-900 mg, or in a daily dose of 200-800 mg, or in a daily dose of 200-600 mg, or in a daily dose of 400-900 mg, or in a daily dose of 400-600 mg.

8. The pharmaceutical composition for use according to any one of claims 2-7, wherein 28 days constitute one treatment cycle, and the total dose of the pharmaceutical composition comprising the compound of formula I or the pharmaceutically acceptable salt thereof administered on days 1-28 in each treatment cycle is 2.8 g-33.6 g, 5.6 g-25.2 g, 11.2 g-16.8 g, or 11.2 g-25.2 g.

9. The pharmaceutical composition for use according to any one of claims 2-8, wherein 21 days constitute one treatment cycle, and the total dose of the pharmaceutical composition comprising the compound of formula I or the pharmaceutically acceptable salt thereof administered on days 1-21 in each treatment cycle is 2.1 g-25.2 g, 4.2 g-18.9 g, 8.4 g-12.6 g, or 8.4 g-18.9 g.

10. The compound of formula I or the pharmaceutically acceptable salt thereof, or the pharmaceutical composition for use according to any one of claims 1-9, wherein the compound of formula I or the pharmaceutically acceptable salt thereof, or the pharmaceutical composition is used in combination with an additional chemotherapeutic drug, and the additional chemotherapeutic drug comprises a pyrimidine anti-tumor drug, a fluorouracil anti-tumor drug, a platinum-based anti-tumor drug, a paclitaxel anti-tumor drug, a tyrosine kinase inhibitor, a PD-1/PD-L1 inhibitor, a camptothecin anti-tumor drug, a BRAF inhibitor, or any combination thereof;
preferably, the additional chemotherapeutic drug is a fluorouracil anti-tumor drug, a platinum-based anti-tumor drug, or a combination thereof;
more preferably, the additional chemotherapeutic drug is a combination of capecitabine and oxaliplatin.

11. The compound of formula I or the pharmaceutically acceptable salt thereof, or the pharmaceutical composition for use according to any one of claims 1-10, wherein the biliary tract cancer is intrahepatic cholangiocarcinoma, extrahepatic cholangiocarcinoma, or gallbladder cancer; or the cholangiocarcinoma is cholangiocellular carcinoma.

12. The compound of formula I or the pharmaceutically acceptable salt thereof, or the pharmaceutical composition for use according to claim 11, wherein the biliary tract cancer is biliary tract cancer with an IDH1 gene mutation; preferably, the IDH1 gene mutation comprises one or more of R132C, R132G, R132H, R132L, or R132S mutation.

13. The compound of formula I or the pharmaceutically acceptable salt thereof, or the pharmaceutical composition for use according to claim 11 or 12, wherein the biliary tract cancer is locally advanced, recurrent, and/or metastatic biliary tract cancer;
preferably, the biliary tract cancer is locally advanced, recurrent, and/or metastatic biliary tract cancer that has not previously been treated systemically for advanced disease.

14. The compound of formula I or the pharmaceutically acceptable salt thereof, or the pharmaceutical composition for use according to any one of claims 11-13, wherein the biliary tract cancer is biliary tract cancer that has previously failed treatment with a gemcitabine, and/or fluorouracil, and/or platinum-based drug; preferably, the biliary tract cancer is locally advanced, recurrent, and/or metastatic biliary tract cancer with an IDH1 mutation that has previously failed treatment with a gemcitabine, and/or fluorouracil, and/or platinum-based drug.

15. Use of a compound of formula I or a pharmaceutically acceptable salt thereof, or a pharmaceutical composition comprising same for preparing a medicament for treating biliary tract cancer in a patient,
